Europäisches Patentamt

European Patent Office

Office européen des brevets

(11) Publication number: **0 036 910**

**A1**

## EUROPEAN PATENT APPLICATION

(21) Application number: 80200253.5

(22) Date of filing: 21.03.80

(51) Int. Cl.³: **A 61 F 7/10**
**A 61 F 7/02**

(43) Date of publication of application:
07.10.81 Bulletin 81/40

(84) Designated Contracting States:
AT BE CH DE FR GB IT LU NL SE

(71) Applicant: Hope, Thomas Clifford Forgan
26 Coonara Street
Holland Park Brisbane 4121(AU)

(71) Applicant: Quintner, Alexander David
19 Welbeck Street
Alderley Heights Brisbane 4051(AU)

(72) Inventor: Hope, Thomas Clifford Forgan
26 Coonara Street
Holland Park Brisbane 4121(AU)

(72) Inventor: Quintner, Alexander David
19 Welbeck Street
Alderley Heights Brisbane 4051(AU)

(74) Representative: Houghton, David et al,
Hulse & Co. Cavendish Buildings West Street
Sheffield, S1 1ZZ(GB)

(54) **A thermal bandage.**

(57) This application relates to a thermal bandage (10) for the treatment of such injuries as sprains, bruises, burns and the like.

In one embodiment the bandage takes the form of a flexible plastic container (11) containing a chillable liquid which will not freeze when stored in a domestic refrigerator and which is segmented to enable the respective compartments (12) to hinge with respect to one another so that the bandage (10) may be secured about a limb by tie means (16, 17) integral with the bandage (10).

FIG I

EP 0 036 910 A1

Croydon Printing Company Ltd.

- 1 -

## A THERMAL BANDAGE

This invention relates to thermal bandages.

It has been established in recent times that the most effective analgesic during the first forty-eight hours following such injuries as bruising, sprains, burns and the like, is an ice pack applied to the painful area. Also, ice packs are useful for treating headaches and stress conditions, while warm packs are useful for treating sinus and other associated nasal conditions.

The present invention has been devised to provide a thermal bandage which may be used for chilling or warming affected of injured areas, which will be simple and effective to use and reliable in operation. Other objects and advantages of this invention will hereinafter become apparent. The present invention also provides a thermal bandage which may be used in the treatment of animals.

With the foregoing and other objects in view, this invention resides broadly, in one aspect, in a thermal bandage including a flexible container having sealed therewithin a liquid medium having a low freezing point whereby the bandage will remain flexible at low temperatures and will, when placed against a patient's body, substantially conform to the shape thereof.

In order that the invention may be more readily understood and put into practical effect, reference will now be made to a preferred embodiment of the invention, wherein:-

Fig. 1 illustrates a thermal bandage according to the present invention, particularly suitable for the treatment of injured limbs;

Fig. 2 illustrates the application of a face mask according to the present invention; and

Fig. 3 illustrates a further embodiment of the invention for use on animals and the like.

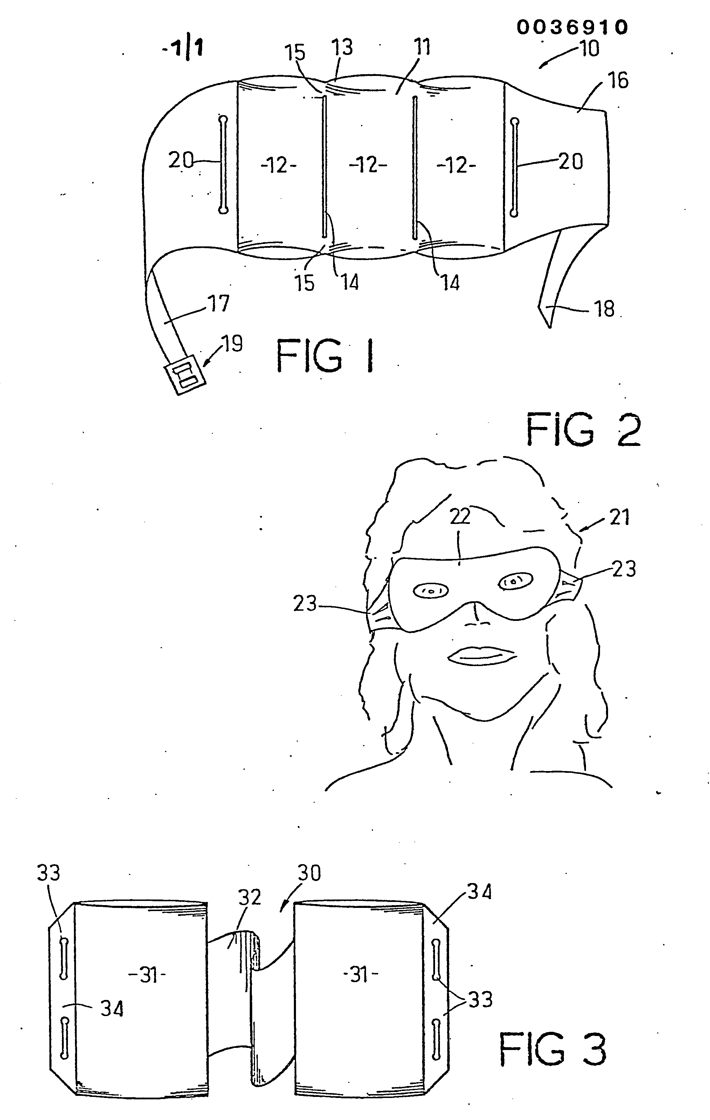

Referring to Fig. 1, there is shown a thermal bandage 10 adapted to be secured about the limb of a person for the treatment of say a sprain or the like and comprising a fluid-filled substantially rectangular package-like body portion 11 suitably 25 cm. long by 15 cm. deep. The body portion is formed as a fluid-filled chamber formed between flexible plastic skins which are sealed along their peripheral edges 13 and which are connected sealably together at transverse zones as at 14 so as to divide the body portion into three compartments 12. Each compartment is able to communicate with the adjacent compartment through the gap 15 at the end of each zone 14, each of which is provided to form a hinge joint for the fluid-filled body portion so that the bandaging may be bent around a limb or the like, while maintaining substantially even distribution of fluid in each compartment 12. If the hinge zones 14 were not provided the body part would flatten at the bent zone and the amount of chilled medium at that zone would be reduced significantly so that the effect of the thermal treatment would not be provided effectively at that point.

Securing straps 16 and 17 extend taperingly away from the respective opposite ends of the body part 11 to enable the bandage to be quickly secured about a limb or the like, and for this purpose one strap 16 terminates with a plane parallel tail portion 18 while the other strap is provided with a buckle assembly 19 through which the tail 18 may be secured and selectively adjusted to suit the particular application. Additional transversely extending slots 20 are provided adjacent the connection between the straps 16 and 17 of the body part 11 to enable external packaging or bandaging to be inserted through the slots as desired depending upon the particular limb to be treated.

Preferably, the fluid in the package-like body portion 11 is a solution comprising a mixture of water plus 20% by volume glycerol, a small amount of dye - for example Methylene Blue, an anti-bacterial agent, suitably Alkyldimrthylbenzylammonium Chloride and a suitable silicone type anti-foaming agent such as that known as "Silicone A.M. 439". The contained liquid in accordance with the above mixture will have good thermal properties for the purposes of the invention and when contained in the clear plastic package will have a pleasing soothing appearance. The anti-bacterial agent is added to prevent the growth of algae and the like on the inside of the clear package so that the pleasing and soothing appearance of the package will be retained while an anti-foam agent has been added to enable the package to be filled with the liquid by an injection process without the inclusion in the package of a large amount of entrapped air and/or other gases.

In the embodiment illustrated in Fig. 2, the invention is applied to a face mask 21 comprising a body portion 22 shaped to fit neatly about the wearer's eyes as shown and containing a chillable liquid as in the previous embodiment. Side straps 23 which extend away from the body portion may be tied at the back of the head to secure the pack about the eyes forwardly of the wearer. This mask is designed for the relief of headaches and stress due to tiredness and heat and for this purpose it is pre-chilled prior to use. The mask may be used also·at an elevated temperature for the relief of sinus and other nasal disorders.

The present invention also provides a thermal bandage 30 which is particularly suitable for the treatment of sprains and the like in large animals such as horses. In this embodiment, the bandage comprises a pair of liquid-filled compartments 31, each of which may be segmented if desired, and operatively joined by a flexible connection panel 32. Securing slots 33 are formed in an extension flap portion 34 provided at the opposite extremities of the package. The flexible connector panel 32 is provided so that the package may be inserted for example about the leg of an animal and secured thereto with the liquid containing portions of the bandage in direct contact with the injured portion of the leg. For this purpose, the connector panel may be fully extended when being utilised to treat a large animal or the panel 32 may be doubled over upon itself to enable the liquid-filled compartments to be placed close together or to enable the bandage 30 to be secured about the limb of a small animal.

In use, the thermal bandage according to this invention may be kept in the freezer ready for use or it may be transported to a sporting venue, where sprains and the like are commonplace, in the insulated container used to carry the refreshments for the players. The composition of the fluid is such that its freezing point is below zero so that it will remain flexible even though it has been stored in a freezer or the like. The bandage is, of course, then close to hand ready for immediate application. For the treatment of sinus and the like condition, the bandage is heated in a saucepan of water to the desired temperature prior to use. Of course, a face mask may be maintained in a refrigerator rather than a freezer so that it will not cause discomfort to the patient.

It will of course be realised that the above has been given by way of illustrative example only and that many modifications and variations as would be apparent to persons skilled in the art are deemed to fall within the broad scope and ambit of the invention as is defined in

0036910

the appended claims.

1.      A thermal bandage including a flexible container having sealed therewithin a liquid medium having a low freezing point whereby the bandage will remain flexible at low temperatures and will, when placed against a patient's body, substantially conform to the shape thereof.

2.      A thermal bandage according to Claim 1, wherein said liquid medium has a freezing point below $0^{o}C$ whereby said bandage may be stored ready for use in a domestic freezer.

3.      A thermal bandage according to Claim 1 or Claim 2, wherein said liquid is a mixture of water and glycerol.

4.      A thermal bandage according to any one of the preceding claims wherein glycerol constitutes substantially 20% by volume of said mixture and wherein there is additionally provided in said liquid an anti-bacterial agent and an anti-foaming agent.

5.      A thermal bandage according to Claim 3, wherein the anti-bacterial agent is Alkyldimrthylbenzylammonium Chloride and said anti-foaming agent is "Silicone A.M. 439".

6.      A thermal bandage according to any one of the preceding claims, wherein said flexible container is substantially rectangular and constitutes a main body portion, and wherein strap means extend away from opposite ends of the body portion to enable said bandage to be strapped about a patient.

7.      A thermal bandage according to Claim 5, wherein said body portion is segmented by spaced transversely extending hinges whereby said body portion may be bent about the limb of a patient and substantially conform to the

0036910

shape of the limb.

8.      A thermal bandage according to any one of the preceding claims, wherein the body portion is provided with spaced cutouts for through-viewing when the bandage is applied, mask-like, to a patient's face.

9.      A thermal bandage according to any one of claims 1 to 5, wherein there are provided a pair of said flexible containers connected together by an intermediate flexible panel whereby the bandage may be applied to a patient with the respective containers spaced closely together or widely apart, as desired.

10.      A thermal bandage according to Claim 9, wherein the sides of said flexible containers opposite said flexible panel are provided with apertured flaps to enable a bandage or the like to pass therethrough to secure said bandage to the patient.

0036910

-1/1

FIG 1

FIG 2

FIG 3

# EUROPEAN SEARCH REPORT

European Patent Office

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.³) |
|---|---|---|---|
| X | US - A - 2 697 424 (E.L. HANNA)<br>* column 1, lines 15 to 22 *<br>-- | 1,2,<br>10 | A 61 F 7/10<br>A 61 F 7/02 |
| X | US - A - 2 595 328 (C.T. BOWEN)<br>* column 4, lines 19 to 25 *<br>-- | 1-3,<br>7 | |
| X | US - A- 2 378 087 (J.M. KEARNEY)<br>* claims 1, 2 *<br>-- | 1,2 | |
| X | US - A - 2 366 989 (B.R. ROBERTSON)<br>* entire document *<br>-- | 1-4 | TECHNICAL FIELDS SEARCHED (Int.Cl.³) |
| | DE -U - 1 930 103 (F. STELLING)<br>* fig. 1, 4 *<br>-- | 1,6,<br>7 | A 61 F 7/00 |
| | BE - A - 711 813 (B.T. GLENSFELDT)<br>* fig. 1 *<br>-- | 1,6,<br>8 | |
| | FR - A - 2 063 258 (M.A. DARRICAU)<br>* fig. *<br>-- | 1,6,<br>7 | |
| | GB - A - 1 404 171 (UNION CARBIDE CORP.)<br>* claims 1 to 3 *<br>-- | 1,6 | |
| | US - A - 3 463 161 (S. ANDRASSY)<br>* claim 1; fig. 2, 3 *<br>-- | 1,6,<br>7 | |
| | US - A - 3 075 529 (J.V. YOUNG, JR.)<br>* claim 1 *   -- ./.. | 1,7 | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 05-01-1981 | KANAL |

EPO Form 1503.1  06.78

0036910

European Patent Office

**EUROPEAN SEARCH REPORT**

Application number

EP 80 20 0253.5
- page 2 -

| Category | Citation of document with indication, where appropriate, of relevant passages | Relevant to claim |
|---|---|---|
| DOCUMENTS CONSIDERED TO BE RELEVANT ||| CLASSIFICATION OF THE APPLICATION (Int. Cl.³) |
| A | DE - C - 629 448 (A. BLUM)<br>* fig. 1 to 4 * | 1,7,<br>10 | |
| A | FR - A - 1 365 666 (H. LOINTIER et al.)<br>* fig. 4, 7, 8 * | 1,6,<br>7 | |

TECHNICAL FIELDS SEARCHED (Int. Cl.³)